# EUROPEAN PATENT APPLICATION

(11) **EP 2 119 779 A1**
(43) Date of publication of application: **18.11.2009**
(21) Application number: 08703341.1
(22) Date of filing: 17.01.2008
(51) Int. Cl.: C12N 15/09, A01H 1/00, A01H 5/00, C07K 14/415, C12N 5/10

(54) **CONVENIENT METHOD FOR INHIBITION OF GENE EXPRESSION USING RSIS**

(30) Priority: 18.01.2007 JP 2007008994
(71) Applicant: NATIONAL INSTITUTE OF AGROBIOLOGICAL SCIENCES, Tsukuba-shi, Ibaraki 305-8602 (JP)
(72) Inventor: TAKAIWA, Fumio, Tsukuba-shi Ibaraki 305-8602 (JP); YASUDA, Hiroshi, Tsukuba-shi Ibaraki 305-8602 (JP)
(74) Representative: Bösl, Raphael Konrad
(86) International application number: PCT/JP2008/050481
(87) International publication number: WO 2008/087998

(57) **Abstract**

The present inventors identified RSIS, and succeeded in suppressing the expression of a target gene by linking RSIS between the region from the promoter to mRNA 5' end sequence of a target gene and a terminator sequence including the 3'UTR after the stop codon. Furthermore, the inventors demonstrated that the expression of two different genes could be suppressed at the same time in cells where a promoter was active, by using the promoter and terminator derived from different genes, and a portion of the mRNA of each gene.

## Description

### Technical Field

The present invention relates to DNAs that induce RNA silencing; DNAs comprising such a DNA, which suppress the expression of a target gene; simple methods for suppressing the expression of a target gene or inducing the silencing of a target gene by using such a DNA; agents for suppressing the expression of a target gene or inducing the silencing of a target gene; and methods for producing siRNA expression vectors.

### Background Art

The genomic sequences of rice and *Arabidopsis,* which are regarded as model plants, have been almost completely determined. Elucidation of the functions of their gene products (proteins) will be an important objective to be achieved in the future. There are carious methods for elucidating protein functions, and two types of genetic methods are used to shed light on *in vivo* functions.

The first method is a forward genetic method based on mutant (phenotypical abnormality) screening and identification of causative genes. Mutants are generated by mutagen treatment, γ-ray irradiation, etc.

The second method is a reverse genetic method based on observation of phenotypic alterations following structural disruption or suppression of expression of known genes. In the reverse genetic method, the expression of known genes is controlled by forced expression through gene recombination, knock down through homologous recombination, transposon tagging, or RNA silencing, etc. However, in plants, it is difficult to use homologous recombination as a method for suppressing the expression of known genes, and thus RNA silencing is commonly used.

Due to the development of molecular biology techniques and establishment of transformation methods in plant research, in addition to the field of basic science, for example, to elucidate functions of unknown genes, RNA silencing is now also used in the field of applied science, for example, to add higher value to crops (see Non-patent Documents 1 to 3). Furthermore, artificial RNA silencing is used to develop virus-resistant crops since RNA silencing was demonstrated to be involved in innate virus resistance in plants (see Non-patent Documents 4 and 5). The RNA silencing method is expected to be more frequently used in future as a technique for developing high-value-added crops.

The antisense method has been used until now as a method for suppressing the expression of genes of interest using genetic recombination techniques. In recent years, however, the method using RNA silencing, which is more frequently successful with a greater suppression effect than the antisense method, has become mainstream. The mechanism of RNA silencing is also being revealed. The process of RNA silencing can be roughly divided into the following three steps. The first step is the formation of double-stranded RNA (dsRNA); the second step is the fragmentation into double-stranded RNA of 21 to 26 base pairs (siRNA; see Non-patent Document 7) by Dicer (see Non-patent Document 6); and the third step is degradation of mRNA having a sequence homologous to siRNA by RNA-induced silencing complex (RISC) (see Non-patent Document 8).

The most important factor for inducing artificial RNA silencing is formation of double-stranded RNA. A known method for double-stranded RNA formation, which can efficiently induce RNA silencing, comprises allowing the transcript to form an inverted repeat or hairpin-loop structure. Furthermore, it is also known that the silencing efficiency can be further improved by using intron sequences as a spacer (see Non-patent Documents 9 and 10). In addition, vectors (pHANNIBAL, pHELLSGATE, and pANDA) that enable the formation of such a structure have also been developed (see Non-patent Documents 11 and 12).

One finding on RNA silencing in rice plants uses the mGLP-1 sequence, in which the codons of the nucleotide sequence of glucagon-like peptide 1 (GLP-1), a human peptide hormone, have been replaced with codons that appear frequently in the rice endosperm (see Non-patent Document 13). However, this mGLP-1 sequence is not suitable for practical use, because it contains multiple restriction sites. Furthermore, glutelin gene suppression by the mGLP-1 expression is considered to be merely a special phenomenon.
Non-patent Document 1: Fukusaki E. et al. Flower color modulations of Torenia hybrida by downregulation of chalcone synthase genes with RNA interference. J. Biotechnol. 111: 229-240 (2004).
Non-patent Document 2: Liu Q. et al. High-stearic and high-oleic cottonseed oils produced by hairpin RNA-mediated post-transcriptional gene silencing. Plant Physiol. 129; 1732-1743 (2002).
Non-patent Document 3: Ogita S. et al. Producing decaffeinated coffee plants. Nature 423; 823 (2003).
Non-patent Document 4: Lindbo J. A. et al. Induction of a highly specific antiviral state in transgenic plants: implications for regulation of gene expression and virus resistance. Plant Cell 5; 1749-1759(1993).
Non-patent Document 5: Waterhouse P. M. et al. Virus resistance and gene silencing in plants can be induced by simultaneous expression of sense and antisense RNA. Proc. Natl. Acad. Sci. USA 95; 13959-13964 (1998).
Non-patent Document 6: Bernstein E. et al. Role for a bidentate ribonuclease in the initiation step of RNA interference. Nature 409; 363-366 (2001).
Non-patent Document 7: Hamilton A. J. and Baulcombe D. C. A species of small antisense RNA in posttranscriptional gene silencing in plants. Science 286; 950-952 (1999).
Non-patent Document 8: Rand T.A. et al. Biochemical identification of Agronaute 2 as the sole protein required for RNA-induced silencing complex activity. Proc. Natl. Acad. Sci. USA 101; 14385-14389(2004).
Non-patent Document 9: Wesley S. V. et al. Construct design for efficient, effective and high-throughput gene silencing in plants. Plant J. 27; 581-590 (2001).
Non-patent Document 10: Smith N. A. et al. Total silencing by intron-spliced hairpin RNAs. Nature 407; 319-320 (2000).
Non-patent Document 11: Helliwell C. and Waterhouse P. Constructs and methods for high-throughput gene silencing in plants. Methods 30; 289-295 (2003).
Non-patent Document 12: Miki D. and Shimamoto K. Simple RNAi vectors for stable and transient suppression of gene function in rice. Plant Cell Physiol. 45; 490-495 (2004).
Non-patent Document 13: Yasuda H. et al. Expression of the small peptide GLP-1 in transgenic plants. Transgenic Research 14; 677-684 (2005).

### Disclosure of the Invention

### [Problems to be Solved by the Invention]

An objective of the present invention is to provide DNAs that induce RNA silencing; DNAs comprising such a DNA, which suppress the expression of a target gene; simple methods for suppressing the expression of a target gene or inducing the silencing of a target gene by using such a DNA; agents for suppressing the expression of a target gene or inducing the silencing of a target gene; and methods for producing siRNA expression vectors.

### [Means for Solving the Problems]

The present inventors conducted dedicated studies to achieve the above-described objective.

They succeeded in identifying a sequence capable of inducing RNA silencing (RNA silencing inducible sequence (RSIS)) in the rice endosperm. The expression of target genes could be suppressed by linking the sequence to the target genes between the region from the promoter and the 5' end sequence of the mRNA (about 100 bp) and the terminator sequence after the stop codon.

The present inventors also demonstrated that, by using a promoter and terminator originating from two different genes, the expression of the two genes could be suppressed at the same time in cells where the promoter was active. The present inventors further demonstrated that the RNA silencing signal induced in the rice endosperm was not transduced to the embryos of rice seeds, and also that RSIS-mediated RNA silencing could be induced in the rice leaf.

In 2005, the present inventors performed RNA silencing in rice plant using the mGLP-1 I sequence (see Non-patent Document 13). However, the mGLP sequence contains multiple restriction sites, which was very disadvantageous to prepare constructs (vectors) and thus the sequence was not suitable for practical use. In this invention, the present inventors succeeded in discovering a more practical sequence RSIS, by deleting all the restriction sites in mGLP. This RSIS makes it very easy to prepare constructs (vectors), and thus has acquired markedly improved practical utility. Furthermore, the present inventors confirmed that the silencing efficiency remained unaltered after deleting the restriction sites. The present inventors also demonstrated that the silencing could be induced with the same efficiency even when the translation frame was shifted. At the time the present inventors published Non-patent Document 13 in 2005, glutelin gene suppression by the mGLP-1 expression was thought to be merely a special phenomenon. The present invention, however, demonstrates that RSIS can be commonly used to suppress the expression of not only glutelin but also other genes.

Furthermore, the RSIS-based method for suppressing the expression of genes does not need preparation of constructs with complicated conformations, and is expected to be especially effective in suppressing the expression of multiple genes.

As described above, the present inventors succeeded in suppressing the expression of target genes by using RSIS, and thus completed the present invention. The expression of target genes can be controlled by using RSIS.

Specifically, the present invention relates to DNAs that induce RNA silencing; DNAs comprising such a DNA, which suppress the expression of a target gene; simple methods for suppressing the expression of a target gene or inducing the silencing of a target gene, which use such a DNA; agents for suppressing the expression of a target gene or inducing the silencing of a target gene; and methods for producing siRNA expression vectors. More specifically, the present invention relates to:
[1] a DNA of any one of:
   (a) a DNA comprising the nucleotide sequence of SEQ ID NO: 3;
   (b) a DNA that hybridizes under stringent conditions to a DNA comprising the nucleotide sequence of SEQ ID NO: 3 and is functionally equivalent to the DNA comprising the nucleotide sequence of SEQ ID NO: 3; and
   (c) a DNA comprising a nucleotide sequence having a substitution, deletion, addition, and/or insertion of one or more nucleotides in the nucleotide sequence of SEQ ID NO: 3;
[2] a DNA that suppresses the expression of a target gene, which has a structure in which the DNA of [1] is operably linked to:
   (a) a DNA encoding a promoter sequence upstream of the DNA of [1];
   (b) a DNA encoding a terminator sequence downstream of the DNA of [1]; and
   (c) a DNA encoding the full-length or a partial mRNA of the target gene downstream of the DNA of (a) and upstream of the DNA of (b);
[3] a DNA that suppresses the expression of multiple target genes at the same time, which has a structure in which the DNA of [1] is operably linked to:
   (a) a DNA encoding a promoter sequence upstream of the DNA of [1];
   (b) a DNA encoding a terminator sequence downstream of the DNA of [1]; and
   (c) a DNA encoding the full-length or a partial mRNA of each of the multiple target genes downstream of the DNA of (a) and upstream of the DNA of (b);
[4] a protein encoded by the DNA of any one of [1] to [3];
[5] a vector carrying the DNA of any one of [1] to [3];
[6] a composition comprising the DNA of any one of [1] to [3] or the vector of [5];
[7] a transformed plant cell introduced with the DNA of any one of [1] to [3] or the vector of [5];
[8] a transformed plant comprising the transformed plant cell of [7];
[9] a transformed plant, which is a progeny or clone of the transformed plant of [8];
[10] a breeding material of the transformed plant of [8] or [9];
[11] a method for suppressing the expression of a target gene, which uses the DNA of any one of [1] to [3] or the vector of [5];
[12] a method for inducing the silencing of a target gene, which uses the DNA of any one of [1] to [3] or the vector of [5];
[13] a method for producing a transformed plant, which comprises the step of introducing a plant cell with the DNA of any one of [1] to [3] or the vector of [5] and regenerating a plant from the plant cell;
[14] a method for producing a plant in which the expression of a target gene is suppressed, or a seed thereof, which comprises the step of expressing the DNA of any one of [1] to [3] or the vector of [5] in a cell of the plant;
[15] an agent for suppressing the expression of a target gene, which comprises as an active ingredient the DNA of any one of [1] to [3] or the vector of [5];
[16] an agent for inducing the silencing of a target gene, which comprises as an active ingredient the DNA of any one of [1] to [3] or the vector of [5];
[17] a method for producing an siRNA expression vector, which comprises the steps of:
   (1) operably linking the DNA of [1] to:
      (a) a DNA encoding a promoter sequence upstream of the DNA of [1];
      (b) a DNA encoding a terminator sequence downstream of the DNA of [1]; and
      (c) a DNA encoding the full-length or a partial mRNA of the target gene downstream of the DNA of (a) and upstream of the DNA of (b); and
   (2) inserting the DNA into a vector; and
[18] a method for producing an siRNA expression vector, which comprises the steps of:
   (1) operably linking the DNA of [1] to:
      (a) a DNA encoding a promoter sequence upstream of the DNA of [1];
      (b) a DNA encoding a terminator sequence downstream of the DNA of [1]; and
      (c) a DNA encoding the full-length or a partial mRNA of each of the multiple target genes downstream of the DNA of (a) and upstream of the DNA of (b); and
   (2) inserting the DNA into a vector.

### Brief Description of the Drawings

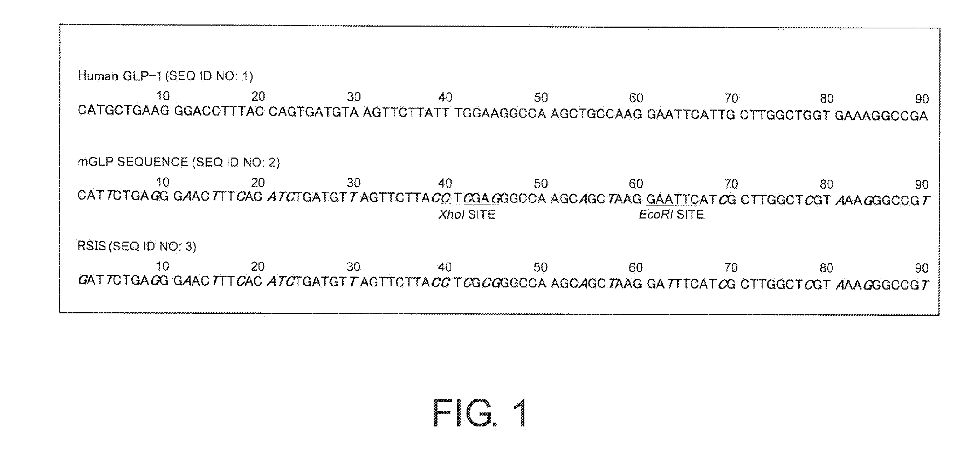

Fig. 1 depicts a diagram showing the nucleotide sequences of mGLP and RSIS. The mGLP sequence (SEQ ID NO: 2) was obtained by replacing the codons of the nucleotide sequence of human GLP-1 (SEQ ID NO: 1) with codons that appear frequently in the rice endosperm. RSIS (SEQ ID NO: 3) was obtained from the mGLP sequence by replacing the *Xho*I and *Eco*RI restriction sites. Bold italics show substituted nucleotide sequences. When compared to human GLP-1, 23 nucleotides (25.6%) have been substituted in the RSIS sequence.

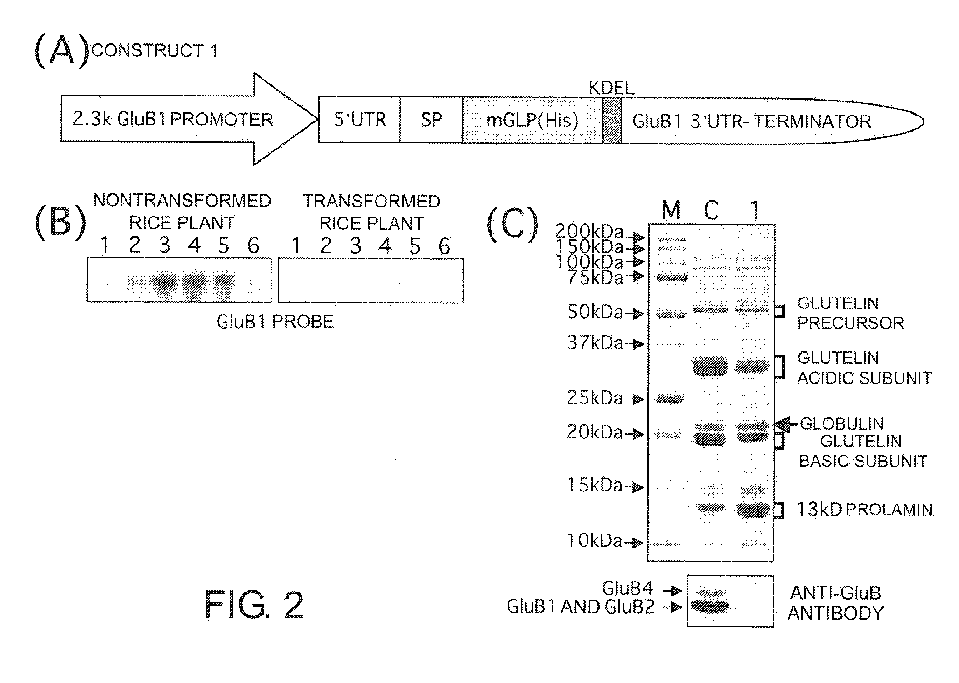

Fig. 2 depicts a diagram and photographs showing the induction of RNA silencing by the mGLP sequence. (A) depicts a schematic diagram showing Construct I for silencing the GluB gene. Construct 1: 2.3-k pGluB1-5'UTR-SP-mGLP (KDEL)-GluB1 3'UTR-terminator. (B) depicts a photograph showing the expression of the GluB1 gene in the seeds of an obtained transformed rice plant. The result of Northern analysis demonstrates that the expression of the GluB1 gene was suppressed in the transformed rice plant (Construct 1). Lane 1, seed 5 days after flowering; lane 2, 7 days after flowering; lane 3, 10 days after flowering; lane 4, 15 days after flowering; lane 5, 20 days after flowering; lane 6, 25 days after flowering. (C) depicts a photograph showing the peptide detection in the seeds of transformed rice plants by SDS-PAGE with an anti-GluB antibody. The result of detection by SDS-PAGE with the anti-GluB antibody showed that the expression of the GluB family was suppressed. In addition, the amounts of globulin and 13-kD prolamin accumulated were found to be increased. Lane M, molecular weight markers; lane C, nontransformed rice plant; lane 1, transformed rice plant (Construct 1).

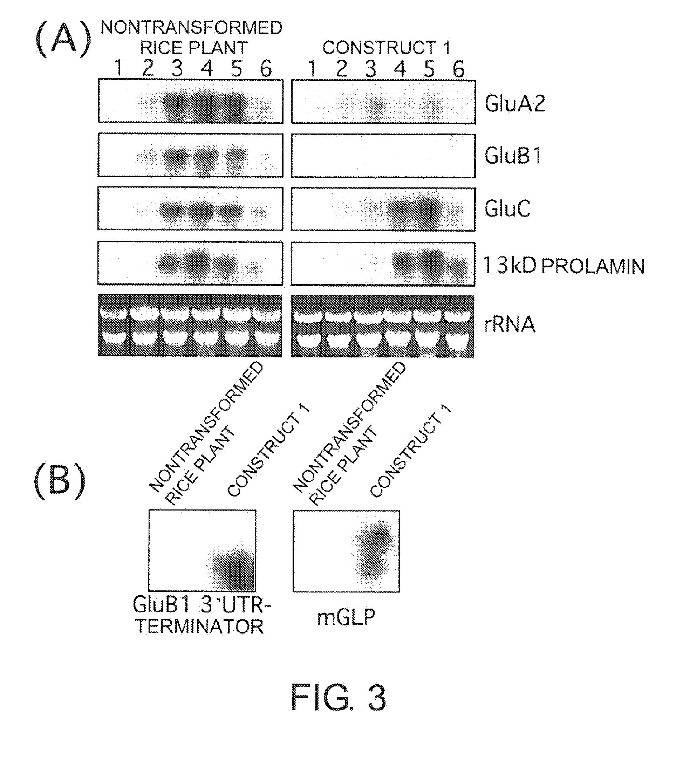

Fig. 3 depicts photographs showing the effect of mGLP sequence-mediated RNA silencing. (A) depicts a photograph showing Northern analysis of seed storage protein in the endosperm of rice plant introduced with Construct 1. The result of Northern analysis showed that the expression of not only the GluB1 gene but also the GluA2 gene, which is highly homologous to the GluB1 gene, was suppressed in the transformed rice plant. By contrast, the expression of 13-kD prolamin and the GluC gene, both of which have low homology, was not suppressed. Lane 1, seed 5 days after flowering; lane 2, 7 days after flowering; lane 3, 10 days after flowering; lane 4, 15 days after flowering; lane 5, 20 days after flowering; lane 6, 25 days after flowering. (B) depicts a photograph showing the detection of siRNA in the endosperm of rice plant introduced with Construct 1. The siRNA was detected in the transformed rice plant (Construct 1) using a probe specific to the introduced gene (mGLP) and a probe specific to the GluB1 gene. This suggests that RNA silencing occurred in the endosperm of transformed rice plant.

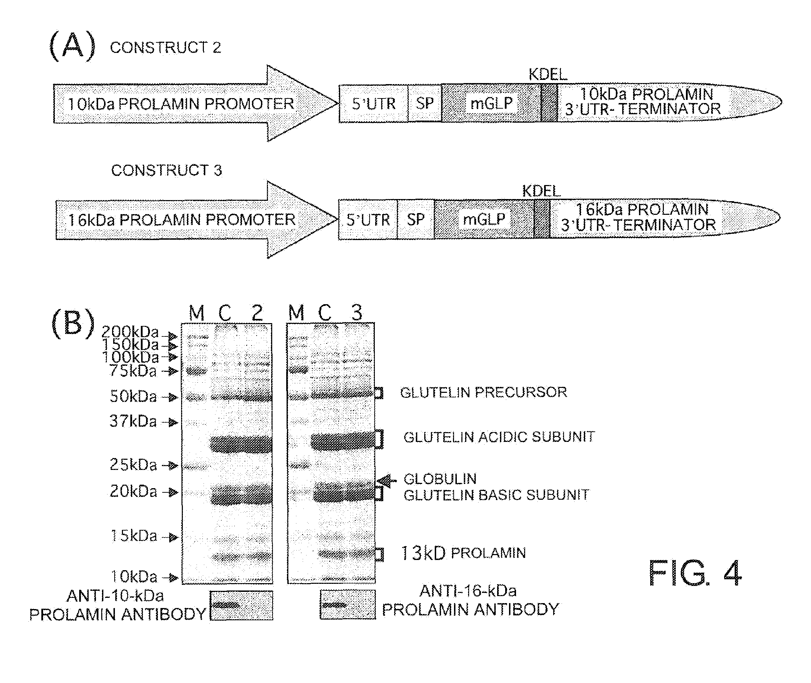

Fig. 4 depicts a diagram and a photograph showing the induction of silencing by the mGLP sequence. (A) depicts a schematic diagram showing Constructs 2 and 3 for silencing the 10-kDa and 16-kDa prolamin genes, respectively. Construct 2, 10-kDa prolamin promoter-5'UTR-SP-mGLP (KDEL)-10-kDa prolamin 3'UTR-terminator; Construct 3, 16-kDa prolamin promoter-5'UTR-SP-mGLP (KDEL)-16-kDa prolamin 3'UTR-terminator. (B) depicts a photograph showing peptide detection in the seed of transformed rice plant by SDS-PAGE with an anti-10k-Da prolamin antibody or anti-16-kDa prolamin antibody. The detection result obtained using the respective antibodies showed that the expression of 10-kDa prolamin (lane 2; Construct 2) and 16-kDa prolamin (lane 3; Construct 3) was suppressed. Lane M, molecular weight markers: lane C, nontransformed rice plant; lane 2, transformed rice plant (Construct 2); lane 3, transformed rice plant (Construct 3).

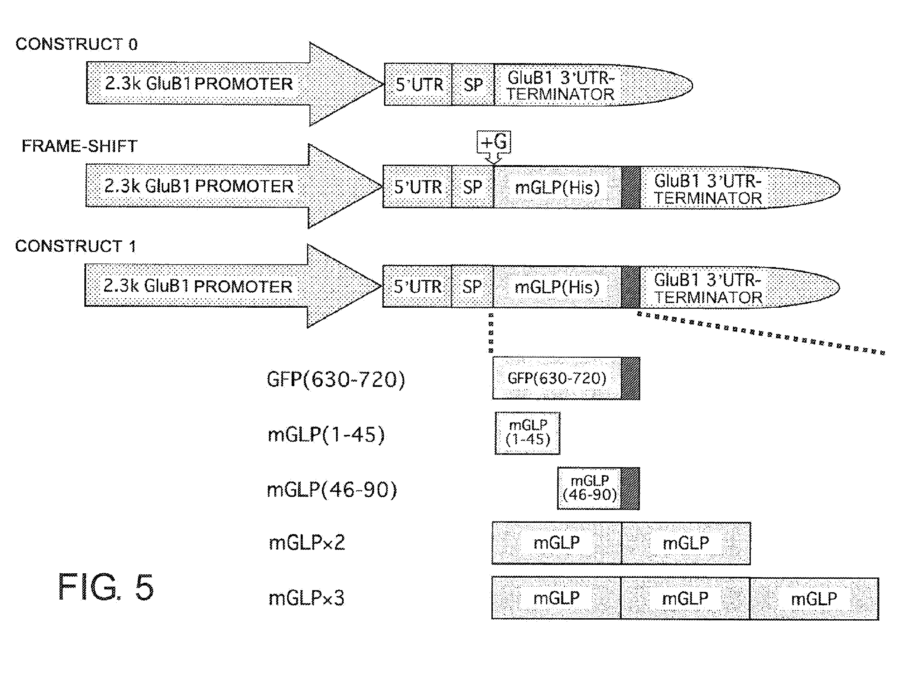

Fig. 5 depicts a diagram showing the constructs that were used to test the activity of the mGLP sequence to induce the silencing.

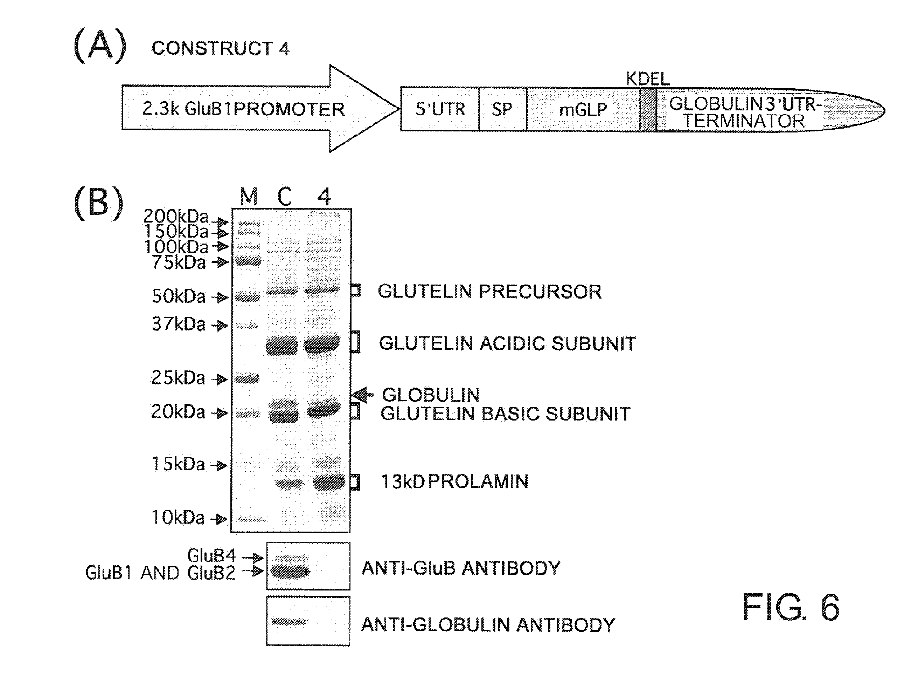

Fig. 6 depicts a diagram and a photograph showing the mGLP sequence-mediated silencing of multiple genes. (A) depicts a schematic diagram showing Construct 4 for simultaneously silencing the GluB and globulin genes. Construct 4, 2.3-k pGluB1-5'UTR-SP-mGLP (KDEL)-globulin 3'UTR-terminator. (B) depicts a photograph showing peptide detection in the seed of transformed rice plant by SDS-PAGE with an anti-GluB antibody or anti-globulin antibody. The detection result obtained by SDS-PAGE with the respective antibodies showed that the expression of the GluB family and globulin was suppressed. In addition, the amounts of glutelins (GluA and GluC) and 13-kD prolamin accumulated were confirmed to be increased. Lane M, molecular weight markers; lane C, nontransformed rice plant; lane 4, transformed rice plant (Construct 4).

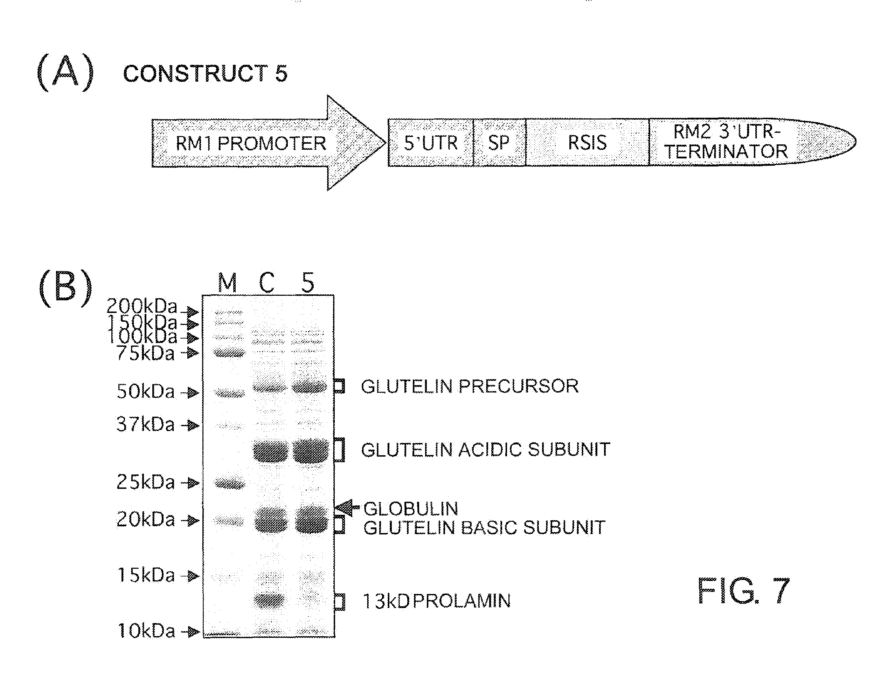

Fig. 7 depicts a diagram and a photograph showing the RSIS sequence-mediated silencing of multiple genes. (A) depicts a schematic diagram showing Construct 5 for silencing the 13-kD prolamin family. Construct 5, RM1 promoter-5'UTR-SP-RSIS-RM2 3'UTR-terminator. (B) depicts a photograph showing a result of SDS-PAGE for the seed of transformed rice plant. It is known that there exist several hundred or more copies of the 13-kD prolamin gene in the rice plant genome. The RM1 and RM2 genes belong to the 13-kD prolamin family. The amount of 13-kD prolamin accumulated was demonstrated to be markedly reduced in the transformed rice plant (Construct 5) in which the RM1 and RM2 genes were silenced with Construct 5 comprising the genes. This suggests that the expression of genes having a similar nucleotide sequence can be suppressed by the mGLP sequence-mediated silencing. Lane M, molecular weight markers; lane C, nontransformed rice plant; lane 5, transformed rice plant 5 (Construct 5).

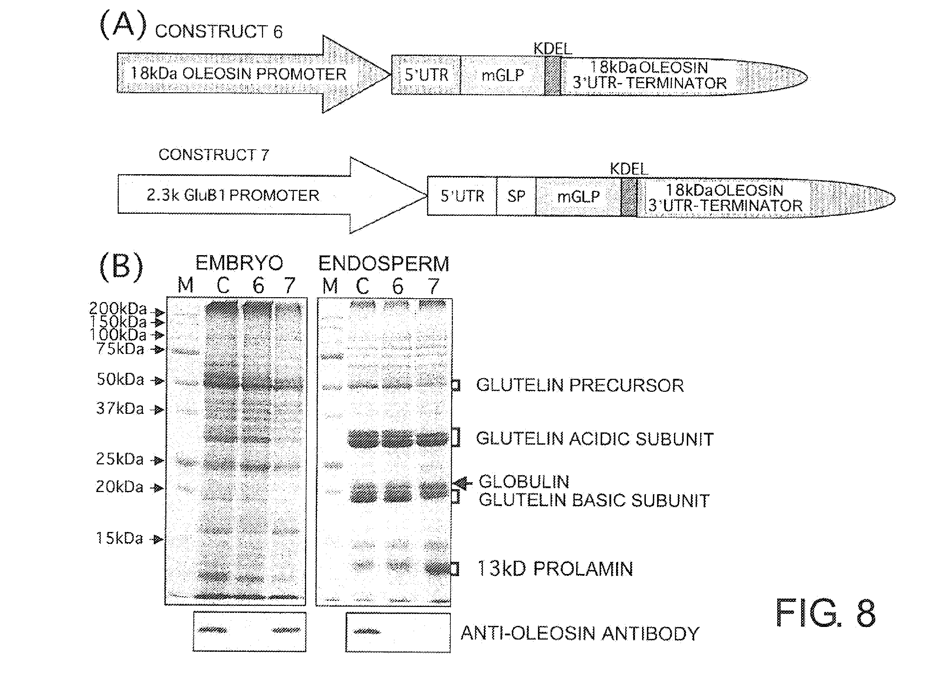

Fig. 8 depicts a diagram and a photograph showing the mGLP sequence-mediated silencing of multiple genes. (A) depicts a schematic diagram showing Constructs 6 and 7 for simultaneously silencing the oleosin and GluB genes. Construct 6, 18-kDa oleosin promoter-5'UTR-mGLP (KDEL)-18-kDa oleosin 3'UTR-terminator; Construct 7, 2.3-k GluB1 promoter-5'UTR-SP-mGLP (KDEL)-18-kDa oleosin 3'UTR-terminator. (B) depicts a photograph showing peptide detection by SDS-PAGE with an anti-oleosin antibody in total proteins prepared from endosperms and embryos of transformed rice plants. The oleosin gene was expressed in the embryo and endosperm. The detection result obtained using the antibody showed that in the transformed rice plant 6 (Construct 6) the expression of the oleosin gene was suppressed in both embryo and endosperm while in the transformed rice plant 7 (Construct 7) the expression was only suppressed in the endosperm. This suggests that the silencing effect is restricted to the expression site of a gene introduced. In transformed rice plant 7, the expression of the CluB gene was also suppressed at the same time. Lane M, molecular weight markers; lane C, nontransformed rice plant; lane 6, transformed rice plant 6 (Construct 6); lane 7, transformed rice plant 7 (Construct 7).

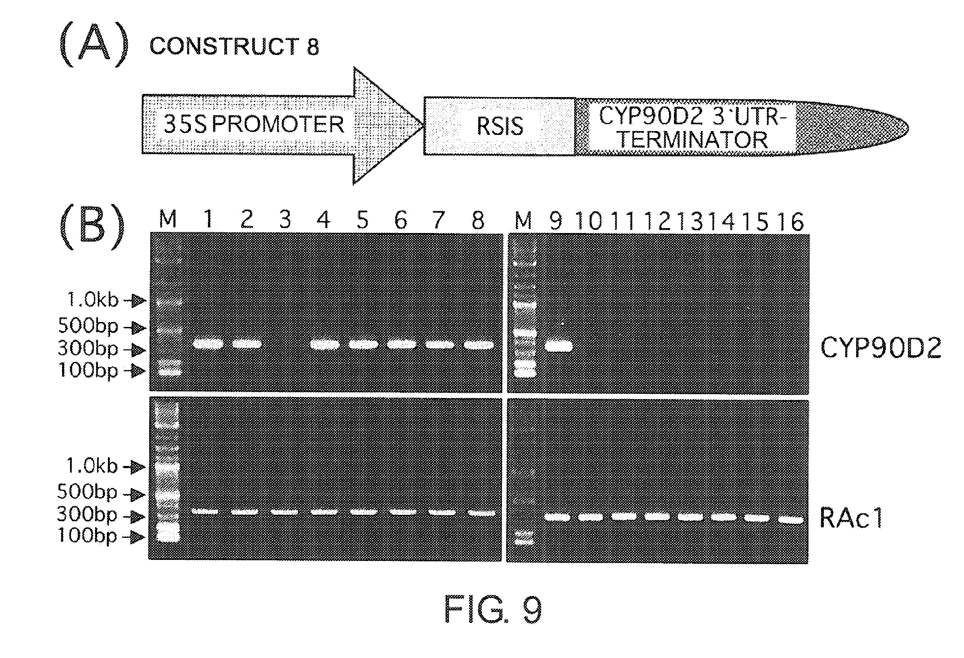

Fig. 9 depicts a diagram and a photograph showing the RSIS-mediated silencing in the leaf blade of rice plant. (A) depicts a schematic diagram showing Construct 8 for silencing the CYP90D2 gene. Construct 8, 35S promoter-RSIS-CYP90D2 3'UTR-terminator. (B) depicts a photograph showing the result of expression analysis for the CYP90D2 gene by RT-PCR. RNA was prepared from the leaf blade of transformed rice plant. After reverse transcription, PCR was carried out using CYP90D2 gene-specific primers. The RAc1 (actin) gene was used as a control. The expression of the CYP90D2 gene was revealed to be suppressed in 26 of 40 plants regenerated. The photograph shows the result for 16 plants. The expression of the CYP90D2 gene was suppressed in lanes 3 and 10 to 16. This suggests that the mGLP sequence-mediated silencing can be induced in not only rice seeds but also in leaf blade. Lane M, molecular weight markers; lanes 1 to 16, transformed rice plant 8 (Construct 8).

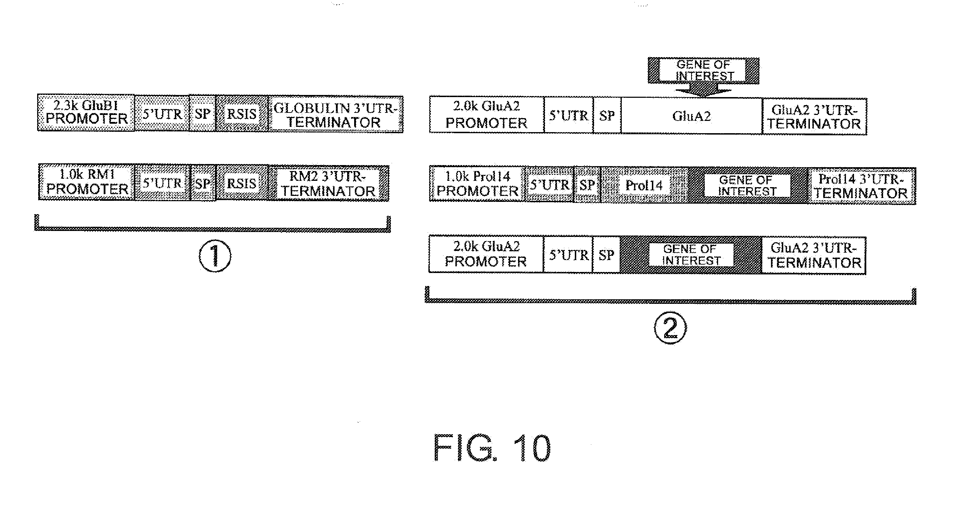

Fig. 10 depicts a diagram showing a method for highly accumulating foreign gene products in rice seeds using RSIS sequence-mediated silencing. The expression of endogenous rice seed storage protein is suppressed with Construct (1), while the foreign gene products are expressed with Construct (2). When Constructs (1) and (2) are used in such a combination that they do not interfere with each other, one can expect to obtain plant lines in which foreign gene products are accumulated in a higher amount as compared to when Construct (2) is introduced alone. The time and effort can be reduced by introducing both constructs by the Gateway system or the like, as compared to the crossing methods, etc. "Prol14" is a kind of 13-kD prolamin. The numbers 1 and 2 marked with circle in this figure are replaced with (1) and (2), respectively in this specification.

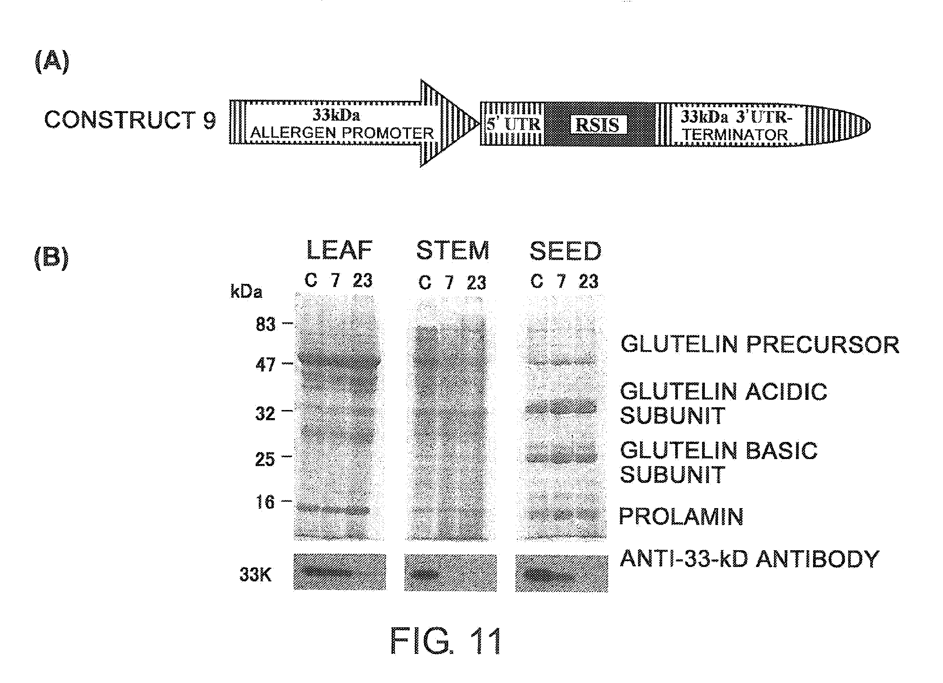

Fig. 11 depicts a diagram and a photograph showing the RSIS sequence-mediated silencing in the leaf, stem, and seed of rice plant. (A) depicts a schematic diagram showing Construct 9 for silencing the rice 33-kDa allergen gene. Construct 9, 33-kDa allergen promoter-5'UTR-RSIS-33-kDa 3'UTR-terminator. (B) depicts a photograph showing a peptide detection result obtained by SDS-PAGE with an anti-33-kDa allergen antibody in total proteins prepared from leaves, stems, and seeds of transformed rice plants. Normally, the 33-kDa allergen gene is expressed in leaves, stems and seeds. The result of detection using the antibody showed that the expression was suppressed in all of leaves, stems, and seeds of transformed rice plants 7 and 23 (both are transformed with Construct 9). The numbers 7 and 23 correspond to the line numbers for the respective transformed plants. Transformed plants with different expression levels were used. Lane C, nontransformed rice plant; lane 7, transformed rice plant (Construct 9); lane 23, transformed rice plant (Construct 9).

### Best Mode for Carrying Out the Invention

The present inventors discovered, for the first time, a sequence capable of inducing RNA silencing (RNA silencing inducible sequence (RSIS)). RNA silencing refers to an RNA-based method for suppressing/inhibiting the expression of a target gene. For example, RNA interference (RNAi) is one type of RNA silencing. RNAi is a phenomenon in which the expression of a target gene is suppressed by inducing the disruption of the target gene mRNA. Conventionally, the disruption of a target gene mRNA is achieved by introducing cells or such with a double-stranded RNA that comprises a sense RNA comprising a sequence homologous to the target gene mRNA, and an antisense RNA comprising a sequence complementary thereto. RNAi can suppress the expression of target genes, and thus is currently a major gene knockout method, surpassing conventional gene disruption methods based on complicated and inefficient homologous recombination. Nucleic acids with inhibitory activity based on the RNAi effect are generally referred to as short interfering RNAs (siRNAs).

The present invention provides novel DNAs that encode RSIS. The nucleotide sequence of RSIS identified by the present inventors is shown in SEQ ID NO: 3.

The present invention also provides DNAs that are functionally equivalent to the above-described DNA comprising the nucleotide sequence of SEQ ID NO: 3. Herein, "functionally equivalent" means that a DNA of interest has biological properties identical to those of the DNA identified by the present inventors. The biological properties of a DNA comprising the nucleotide sequence of SEQ ID NO: 3 include the activity of inducing the silencing of target gene RNA.

Accordingly, those skilled in the art can test whether a DNA of interest induces RNA silencing of a target gene to assess whether the DNA of interest has biological properties identical to those of the DNA comprising the nucleotide sequence of SEQ ID NO: 3 identified by the present inventors.

Organisms for isolating such DNAs include, for example, plants and animals. Such plants include, for example, monocotyledons and dicotyledons plants. Specifically, the plants include, for example, rice plant, *Arabidopsis,* and tobacco, but are not limited thereto.

The DNAs of the present invention also include DNAs that hybridize under stringent conditions to a DNA comprising the nucleotide sequence of SEQ ID NO: 3.

DNAs comprising a nucleotide sequence significantly homologous to the nucleotide sequence of SEQ ID NO: 3 of the present invention can be prepared, for example, by using hybridization techniques (Current Protocols in Molecular Biology edit. Ausubel et al. (1987) Publish. John Wiley & Sons Section 6.3-6.4) and gene amplification techniques (PCR) (Current protocols in Molecular Biology edit. Ausubel et al. (1987) Publish. John Wiley & Sons Section 6.1-6.4). Specifically, based on the nucleotide sequence of SEQ ID NO: 3 or portions thereof, DNAs highly homologous thereto can be isolated from DNA samples derived from the same or a different species of organism, using hybridization techniques. Alternatively, DNAs highly homologous to the nucleotide sequence of the DNA can be isolated using gene amplification techniques by designing primers based on a portion of the nucleotide sequence of SEQ ID NO: 3. Accordingly, the present invention includes DNAs that hybridize under stringent conditions to a DNA comprising the nucleotide sequence of SEQ ID NO: 3.

Such stringent hybridization conditions can be appropriately selected by those skilled in the art. For example, pre-hybridization is carried out at 42°C overnight using a hybridization solution containing 25% formamide, or 50% formamide for more stringent conditions, and 4x SSC, 50 mM Hepes (pH 7.0), 10x Denhardt's solution, and 20 µg/ml denatured salmon sperm DNA. Then, a labeled probe is added to the solution and hybridization is carried out by incubation at 42°C overnight. Post-hybridization washes are carried out under the conditions of about "2x SSC, 0.1% SDS, 50°C", "2x SSC, 0.1% SDS, 42°C", or "1x SSC, 0.1% SDS, 37°C", more stringently "2x SSC, 0.1% SDS, 65°C" or "0.5x SSC, 0.1% SDS, 42°C", or even more stringently "0.2x SSC, 0.1% SDS, 65°C". As the hybridization conditions get stricter, DNAs with higher homology to the probe sequence are expected to be isolated. The above combinations of conditions for SSC, SDS, and temperature are only examples, and those skilled in the art can achieve stringencies equivalent to the above by appropriately combining the above or other factors determining hybridization stringency (for example, probe concentration, probe length, hybridization reaction time, etc.).

The "high homology" refers to at least 50% or higher sequence identity, more preferably 70% or higher sequence identity, and still more preferably 90% or higher sequence identity (for example, 95%, 96%, 97%, 98%, or 99%) over the entire nucleotide sequence.

Such nucleotide sequence identity can be determined using the BLAST algorithm by Karin and Altschul (Proc. Natl. Acad. Sci. USA, 87: 2264-2268 (1990); Karlin, S and Altschul, SF, Proc Natl Acad Sci USA, 90: 5873). A program called BLAST has been developed based on the BLAST algorithm (Altschul, SF et al., J Mol Biol, 215: 403 (1990)), When nucleotide sequences are analyzed using BLASTN, parameters may be set to, for example, score= 100 and wordlength= 12. When the BLAST or Gapped BLAST program is used, default parameters for each program are used. Specific procedures for these analytical methods are known (http://www.ncbi.nlm.nih.gov/).

Furthermore, the present invention includes not only DNAs comprising the nucleotide sequence of SEQ ID NO: 3 but also DNAs comprising a nucleotide sequence having a substitution, deletion, addition, and/or insertion of one or more nucleotides in the nucleotide sequence of SEQ ID NO: 3. Such DNA preparation methods well known to those skilled in the art include, for example, hybridization techniques (Southern, EM., J Mol Biol, 98: 503 (1975)), polymerase chain reaction (PCR) techniques (Saiki, RK. et al., Science, 230: 1350 (1985); Saiki, RK. et al., Science, 239: 487 (1988)), and methods for introducing mutations into the DNA by site-directed mutagenesis (Kramer, W. and Fritz, HJ., Methods Enzymol, 154: 350 (1987)).

In the present invention, the number of nucleotides to be altered by mutations such as deletion and substitution is not particularly limited as long as the DNA introduced with mutations is functionally equivalent to the DNA comprising the nucleotide sequence of SEQ ID NO: 3. The number is typically 20 base pairs or less, preferably 10 base pairs or less, more preferably 5 base pairs or less, and still more preferably 3 base pairs or less. It is within the range of ordinary trial for those skilled in the art to select DNAs functionally equivalent to the DNAs of the present invention from DNAs introduced with mutations as described above.

Nucleotide sequences of the present invention having a substitution, deletion, addition, and/or insertion of one or more nucleotides in the nucleotide sequence of SEQ ID NO: 3 include, for example, the nucleotide sequences of SEQ ID NOs: 1 and 2.

The nucleotide sequence of SEQ ID NO: 1 encodes human GLP-1 protein. The nucleotide sequence of SEQ ID NO: 2 (mGLP-1) is a sequence in which Ser has been substituted for Ala at position 2 in the above-described GLP-1 and codons have been replaced with codons that appear frequently in rice endosperm (furthermore, mGLP-1 encoding an altered mGLP in which Q and D have been substituted for K at positions 20 and 28, respectively, also has the silencing activity; in the report by Yasuda et al. on silencing (Transgenic Res. (2005)), this finding was obtained by using GLP-1 whose amino acid sequence was altered at three positions). The nucleotide sequence of SEQ ID NO: 3 was prepared by deleting some restriction sites (*Xho*I and *Eco*RI sites) from the above-described nucleotide sequence of SEQ ID NO: 2. Specifically, the nucleotide sequence of SEQ ID NO: 3 is prepared by substituting C for A at position 44 and T for A at position 63 in the nucleotide sequence of SEQ ID NO: 2. The nucleotide sequences of SEQ ID NOs: 1 to 3 are shown in Fig. 1.

Specifically, the present invention provides the DNAs described in (a) to (c) below.
(a) a DNA comprising the nucleotide sequence of SEQ ID NO: 3;
(b) a DNA which hybridizes under stringent conditions to a DNA comprising the nucleotide sequence of SEQ ID NO: 3 and is functionally equivalent to the DNA comprising the nucleotide sequence of SEQ ID NO: 3; and
(c) a DNA comprising a nucleotide sequence having a substitution, deletion, addition, and/or insertion of one or more nucleotides in the nucleotide sequence of SEQ ID NO: 3.

The present invention also provides DNAs that suppress the expression of target genes (sometimes referred to as "genes of interest"). Specifically, the present invention provides DNAs having a structure where the DNA of any one of (a) to (c) described above (hereinafter sometimes simply referred to as "DNA encoding RSIS") is operably linked to the DNA of any one of:
(a) a DNA that encodes a promoter sequence upstream of DNA encoding RSIS;
(b) a DNA that encodes a terminator sequence downstream of DNA encoding RSIS; and
(c) a DNA that encodes a full-length or a partial sequence of a target gene mRNA downstream of the DNA of (a) and upstream of the DNA of (b).

The promoter sequence is a sequence that determines the expression timing, level, and site (organ and tissue) of a target gene. Herein, the promoter sequence refers to a sequence upstream of the transcription start site for mRNA, and does not include any portion of mRNA. A sequence from the transcription start site to the translation start site for mRNA is referred to as 5'-untranslated region (5'UTR). In the present invention, the promoter sequence and 5'UTR may be derived from different genes. The promoter sequence used in the present invention may be the promoter sequence of any gene. Such promoters include, for example, the cauliflower mosaic virus 35S promoter sequence, rice actin promoter sequence, and corn ubiquitin promoter sequence.

The promoter also includes, for example, those known to direct the expression by external causes, such as infection or invasion of bacteria or viruses, low temperatures, high temperatures, desiccation, ultraviolet light irradiation, and diffusion of specific compounds. Such promoters include, for example, the promoters of rice chitinase gene and tobacco PR protein gene, which are expressed in response to the infection or invasion of bacteria or viruses; the promoter of rice "lip19" gene induced by low temperature; the promoters of rice "hsp80" and "hsp72" genes induced by high temperatures; the promoter of *Arabidopsis* "rab16" gene induced by desiccation; the promoter of parsley chalcone synthase gene induced by ultraviolet irradiation; and the promoter of corn alcohol dehydrogenase gene induced under an anaerobic condition. Furthermore, the promoter of rice chitinase gene and the promoter of tobacco PR protein gene are also induced by specific compounds, such as salicylic acid, and the promoter of *Arabidopsis* "rab16" gene is also induced in response to the diffusion of a phytohonnone, abscisic acid.

The promoter also includes inducible promoters in animal cells, for example, promoters derived from mammalian cells, and viral promoters such as those derived from cytomegalovirus, retroviruses, polyoma viruses, adenoviruses, and simian virus 40 (SV40).

For example, to suppress the expression of a target gene specifically in the rice endosperm tissue, one may of course use a promoter having an activity specific to rice endosperm tissue.

In the present invention, one of course may use the promoter sequence of a target gene itself.

The terminator sequence (transcription termination sequence) refers to a sequence having the function capable of terminating the transcription initiated from the upstream promoter sequence. Herein, the terminator sequence refers to a sequence downstream of the poly(A) attachment site, and does not include any portion of the mRNA. The sequence from the stop codon to poly(A) attachment site of mRNA is referred to as 3'-untranslated region (3'UTR),

In general, the "terminator sequence" refers to the 3'-untranslated region (3'UTR) from the stop codon to poly(A) attachment site and its downstream region, including a portion of the mRNA. Herein, however, the two regions are distinguished as described above. In the present invention, the gene used as the terminator sequence may be different from the gene used as the 3,UTR.

The terminator sequence used in the present invention includes the terminator sequence of any gene. In the present invention, the terminator sequence includes, for example, terminator sequences comprising the 3'UTR (specifically, sequences called "terminator sequence" in a general sense). The terminator sequence includes, for example, known 3'UTRs such as sequences comprising consecutive four adenine (A) nucleotides and sequences capable of forming a palindromic structure, Such terminator sequences comprising a known 3'UTR include, but are not limited to, for example, terminator sequences comprising a 3'UTR originating from cauliflower mosaic virus, terminator sequences comprising the 3'UTR of octopine synthase, terminator sequences comprising the 3'UTR of the nopaline synthase gene, and terminator sequences comprising the 3'UTR of Agrobacterium.

The terminator sequence of the present invention may of course be the terminator sequence of a target gene itself.

The target gene of the present invention is not particularly limited, and may be any gene. The target gene includes, but is not limited to, for example, genes encoding rice seed storage proteins (glutelin gene, globulin gene, prolamin gene, albumin gene, etc.), brassinosteroid synthase gene (CYP90D2 gene, etc.), genes encoding chaperones (BiP gene and PDI gene), oleosin gene, and 33-kDa allergen gene which is expressed in rice plant in a relatively constitutive fashion.

Furthermore, the present invention uses the full-length or a partial sequence of mRNA of a target gene. Such a partial sequence may originate from any portion of mRNA of the target gene. For convenience, however, it is possible to use 5'-untranslated region (UTR) or 3'-UTR. The length of mRNA to be used is at least 20 nucleotides or more, preferably 100 nucleotides or more, and more preferably 130 nucleotides or more. Such mRNAs include, but are not limited to, for example, the mRNA from 5'-UTR (44 bp) (tcacatcaat tagcttaagt ttccataagc aagtacaaat agct/ SEQ ID NO: 4) to the signal peptide (75 bp) (atggcgagtt ccgttttctc tcggttttct atatactttt gtgttcttct attatgccat ggttctatgg cccag/ SEQ ID NO: 5) of the GluB1 gene, when the target gene is the GluB1 gene.

Herein, "operably linked" means that a DNA encoding RSIS is linked (ligated) to a DNA of any one of (a) to (c) described below, so as to suppress the expression of a target gene:
(a) a DNA that encodes a promoter sequence upstream of a DNA encoding RSIS;
(b) a DNA that encodes a terminator sequence downstream of a DNA encoding RSIS; and
(c) a DNA that encodes the full-length or a partial sequence of target gene mRNA, downstream of the DNA of (a) and upstream of the DNA of (b).
Those skilled in the art can "operably link" readily by using genetic engineering techniques.

In the present invention, a DNA encoding a promoter sequence, a DNA encoding a terminator sequence, a DNA encoding the full-length or a partial sequence of a target gene mRNA, and a DNA encoding RSIS may be linked together as shown below (hereinafter, small hyphen represents a linkage):
[DNA encoding promoter sequence] - [DNA encoding full-length or partial sequence of target gene mRNA] - [DNA encoding RSIS] - [DNA encoding terminator sequence].
   Alternatively, the DNA encoding RSIS maybe linked, for example, as shown below, instead of the linkage indicated above;
[DNA encoding promoter sequence] - [DNA encoding RSIS] - [DNA encoding full-length or partial sequence of target gene mRNA] - [DNA encoding terminator sequence].
Specific linkage examples of the present invention include the linkages shown below which are described in the Examples (Constructs 1 to 3, 6, and 9). In these constructs, the 5'UTR and signal peptide (SP) may be placed between the promoter and the DNA encoding RSIS or the mGLP sequence. Alternatively, a KDEL sequence may be placed between the DNA encoding RSIS or the mGLP sequence, and the terminator. The terminator sequence may comprise a 3'UTR.

[Construct 1] (Fig. 2A)
   [2.3-k GluB1 promoter] - [5'UTR] - = [SP] - [mGLP sequence] - [GluB1 3'UTR terminator]
[Construct 2] (Fig. 4A)
   [10-kDa prolamin promoter] - [5'UTR] - [SP] - [mGLP sequence] - [10-kDa prolamin 3'UTR terminator]
[Construct 3] (Fig. 4A)
   [16-kDa prolamin promoter] - [5'UTR] - [SP] - [mGLP sequence] - [16-kDa prolamin 3'UTR terminator]
[Construct 6] (Fig. 8A)
   [18-kDa oleosin promoter] - [5'UTR] - [mGLP sequence] - [18-kDa oleosin 3'UTR terminator]
[Construct 9] (Fig. 9A.)
   [33-kDa allergen promoter] - [5'UTR] - [RSIS sequence] - [33-kDa allergen 3'UTR terminator]

The present invention also provides DNAs that suppress the expression of multiple target genes at the same time. Specifically, the present invention provides DNAs having a structure where a DNA encoding RSIS is operably linked to:
(a) a DNA that encodes a promoter sequence upstream of a DNA encoding RSIS;
(b) a DNA that encodes a terminator sequence downstream of a DNA encoding RSIS; and
(c) a DNA that encodes the full-length or partial sequences of mRNAs of multiple target genes, downstream of the DNA of (a) and upstream of the DNA of (b).

The promoter sequence of (a) and the terminator sequence of (b) are described above.

Herein, "multiple target genes" means that there are two or more target genes, and preferably the number of target genes is two.

In the present invention, each of mRNAs of the multiple target genes may be the full-length or a partial sequence thereof.

In the present invention, a DNA encoding a promoter sequence, a DNA encoding a terminator] sequence, and a DNA encoding the full-length or a partial sequence of each of mRNAs of multiple target genes, and a DNA encoding RSIS may be linked together as shown below:
[DNA encoding promoter sequence] - [DNA encoding full-length or partial sequence of target gene mRNA] - [DNA encoding RSIS] - [DNA encoding terminator sequence].

For example, when there are two target genes, A and B genes, possible linkages are as follows:
[DNA encoding promoter sequences] - [DNA encoding full-length or partial sequence of A gene mRNA] - [DNA encoding RSIS] - [DNA encoding full-length or partial sequence of B gene mRNA] - [DNA encoding terminator sequence];
[DNA encoding promoter sequence] - [DNA encoding full-length or partial sequence of A gene mRNA] - [DNA encoding full-length or partial sequence of B gene mRNA] - [DNA encoding RSIS] - [DNA encoding terminator sequence]; and
[DNA encoding promoter sequence] - [DNA encoding RSIS] - [DNA encoding full-length or partial sequence of A gene mRNA] - [DNA encoding full-length or partial sequence of B gene mRNA] - [DNA encoding terminator] sequence].

Herein, when 5'UTR or 3'UTR is used independently of [DNA encoding full-length or partial sequence of target gene mRNA], the target gene used as 5'UTR or 3'UTR may be different to or the same as the target gene used as the [DNA encoding full-length or partial sequence of target gene mRNA]. When different target genes are used, the expression of both genes can be suppressed.

Specific linkage examples of the present invention include the linkages shown below (Constructs 4, 5, 7, and 8), which are described in the Examples. In these constructs, the 5'UTR and signal peptide (SP) may be placed between the promoter and the DNA encoding RSIS or the mGLP sequence. Alternatively, a KDEL sequence may be placed between the DNA encoding RSIS or the mGLP sequence and the terminator. The terminator sequence may comprise a 3'UTR.
[Construct 4] (Fig. 6A)
   [2.3-k GluB1 promoter] - [5'UTR] - [SP] - [mGLP sequence] ∼ [globulin 3'UTR-terminator]
[Construct 5] (Fig. 7A)
   [RM1 promoter] - [5'UTR] - [SP] - [DNA encoding RSIS] - [RM2 3'UTR-terminator]
[Construct 7] (Fig. 8A)
   [2.3-k GluB1 promoter] [5'UTR] - [SP] - [mGLP sequence] - [18-kDa oleosin 3 'UTR-terminator]
[Construct 8] (Fig. 9A)
   [35S promoter] - [DNA encoding RSIS] - [CYP90D2 3'UTR-terminator]

Even when there are three or more target genes, DNA encoding the full-length or a partial sequence of each of mRNAs of three or more target genes may be placed together with DNA encoding RSIS between the DNA encoding a promoter sequence and the DNA encoding a terminator sequence.

Hereinafter, the above-described DNAs that suppress the Expression of a target gene or the expression of multiple genes at the same time are sometimes referred to as "DNAs of the present invention".

Furthermore, proteins encoded by the above-described DNAs of the present invention (hereinafter referred to as "proteins of the present invention,") are also included in the present invention.

The present invention also provides vectors carrying DNAs of the present invention as inserts. The vectors of the present invention include vectors for expressing DNAs of the present invention in plant cells, which are used to prepare transformed plant cells or transformed plants. The vectors of the present invention are not particularly limited as long as they stably retain the inserted DNAs. Preferred cloning vectors of the present invention include, for example, pBluescript vector (Stratagene), when the host is *Escherichia coli.*

Those skilled in the art can construct appropriate vectors carrying DNAs of the present invention using conventional genetic engineering techniques. DNAs of the present invention can be inserted into the vectors by conventional methods, for example, by ligation using restriction sites (Current protocols in Molecular Biology edit. Ausubel et al. (1987) Publish. John Wiley & Sons Sections 11.4-11.11). In general, it is possible to use various commercially available vectors.

Such vectors to which the DNAs described above are inserted can be introduced into plant cells by conventional methods known to those skilled in the art, for example, the polyethylene glycol methods, electroporation, the Agrobacterium-mediated method, or the particle gun method. When the Agrobacterium-mediated method is used, the DNAs of the present invention can be introduced into plant cells, for example, by inserting the DNA into an expression vector; introducing the vector into Agrobacteria; and infecting plant cells with the Agrobacteria by direct infection or leaf disc method, according to the method of Nagel et al. (Microbiol. Lett. 67:325 (1990)).

The present invention also provides transformed plant cells introduced with DNAs or vectors of the present invention. The transformed plant cells of the present invention can be in any form, as long as they are plant cells or groups of cells introduced with DNAs or vectors of the present invention and that can regenerate into plants. For example, suspension culture cells, protoplasts, leaf discs, calluses, and such are included in the plant cells of the present invention.

The vectors can be introduced into plant cells by various methods known to those skilled in the art, such as polyethylene glycol methods, electroporation, Agrobacterium-mediated methods, and particle gun methods.

The vectors of the present invention are also useful to maintain DNAs of the present invention in host cells or to express proteins of the present invention in the host cells.

The present invention also provides compositions (mixtures) comprising DNAs or vectors of the present invention. If needed, the compositions of the present invention may comprise, for example, sterile waster, physiological saline, vegetable oils, surfactants, lipids, solubilizing agents, buffer, and preservatives, in addition to DNAs or vectors of the present invention.

The present invention further provides transformed cells introduced with DNAs or vectors of the present invention. Cells to be introduced with DNAs or vectors of the present invention include the above-described cells for producing recombinant proteins and plant cells that are used to produce transformed plants. Such plant cells are not particularly limited, and include, for example, cells of rice, corn, wheat, and barley. The plant cells of the present invention include not only cultured cells but also cells in plants. The plant cells also include protoplasts, shoot primordia, multiple shoots, and hairy roots. The vectors can be introduced into plant cells by various methods known to those skilled in the art, such as polyethylene glycol method, electroporation, Agrobacterium-mediated method, and particle gun method. Plants can be regenerated from plant cell transformants by methods known to those skilled in the art, depending on the plant type. For example, there are already established techniques for producing transformed rice plants that have been widely used in the technical field of the present invention, including the method for introducing genes into protoplasts, using polyethylene glycol and then regenerating plants; the method for introducing genes into protoplasts, using electric pulse and then regenerating plants; the method for directly introducing genes into cells using the particle gun and then regenerating plants; and the method for introducing genes via an Agrobacterium and then regenerating plants. These methods can be appropriately used in the present invention.

In order to efficiently select the cells transformed by introducing a DNA or vector of the present invention, the above-described recombinant vector is introduced into the plant cells, preferably together with a suitable selection marker gene or a plasmid vector comprising a selection marker gene. The selection marker genes used for this purpose include, for example, the hygromycin phosphotransferase gene resistant to the antibiotic hygromycin, the neomycin phosphotransferase gene resistant to kanamycin or gentamycin, and the acetyltransferase gene resistant to the herbicide phosphinothricin.

The plant cells introduced with the recombinant vector are placed on a known selection medium containing a suitable selection agent depending on the type of introduced selection marker gene, and then cultured. Cultured plant cell transformants can be obtained by this procedure.

The transformed plant cells can be regenerated into plants by redifferentiating the cells. The redifferentiation method varies depending on the type of plant cells, and includes, for example, the method of Fujimura et al. (Plant Tissue Culture Lett. 2:74 (1995)) for rice plant, and the methods of Shillito et at. (Bio/Technology 7:581 (1989)) and Gorden-Kamm et al. (Plant Cell 2:603 (1990)) for corn.

Once a transformed plant introduced with a DNA of the present invention into its genome is generated, its progeny can be obtained from the plant via sexual or sexual reproduction. Alternatively, the plant can be produced on a large scale from breeding materials (for example, seeds, fruits, cut panicles, tubers, tuberous roots, sprigs, calluses, and protoplasts) obtained from the plants, its progenies, or their clones. The present invention includes plant cells introduced with a DNA of the present invention; plants comprising the cells; progenies and clones of the plants; and breeding materials of the plants and their progenies and clones.

The expression of target genes is expected to be suppressed in plants produced by the methods described above, or seeds thereof.

Such methods as described above for producing transformed plants, which comprise the step of introducing DNAs or vectors of the present invention into plant cells and regenerating plants from the plants cells, are also included in the present invention.

Furthermore, methods for producing plants and seeds thereof where the expression of target genes is suppressed, which comprise the step of expressing DNAs or vectors of the present invention in cells of plants, are also included in the present invention.

The plants are genetically altered plants in which the expression of target genes has been suppressed artificially. The genetically altered plants are sometimes called, for example, "knockout plants", knockdown plants", or "transgenic plants".

Herein, "the expression of a gene is suppressed artificially" means, for example, that the expression of a target gene is suppressed by the activity of a nucleic acid (for example, siRNA and such) having the function of suppressing the expression of a target gene.

Herein, the "suppression" includes cases where the expression of a target gene is completely suppressed, and cases where the expression level of a target gene in a plant has been significantly reduced as compared to the gene expression level in other plants.

The "nucleic acid" of the present invention refers to "RNA" or "RNA". Furthermore, chemically synthesized nucleic acid analogs such as so-called PNA (peptide nucleic acid) are also included in the nucleic acids of the present invention. PNAs are compounds in which the fundamental backbone structure of nucleic acids, the pentose-phosphate backbone, is replaced by a polyamide backbone configured by glycine units. PNAs have a three-dimensional structure quite similar to that of nucleic acids.

The expression of target genes can be suppressed (or inhibited) in general by RNAi using double-stranded RNAs having sequences identical or similar to the sequences of target genes. RNAs used for RNAi are not necessarily completely identical to target genes or portions thereof; however, they preferably have perfect homology.

In an embodiment, nucleic acids having the function of suppressing the expression of target genes include double-stranded RNAs (siRNAs) having an RNAi effect on the target genes. Of the above-described RNA molecules, those having a closed end, for example, siRNA having a hairpin structure (shRNA) are also included in the present invention. Specifically, single-stranded RNA molecules capable of forming an intramolecular double-stranded RNA structure are also included in the present invention.

According to the present invention, siRNA-based DNA silencing of target genes can be induced by using the above-described DNAs of the present invention.

The present invention also provides methods for suppressing the expression of target genes and methods for inducing target gene silencing, by using DNAs or vectors of the present invention.

The present invention also provides agents for suppressing the expression of target genes and agents for inducing target gene silencing, which comprise as an active ingredient a DNA or vector of the present invention.

Herein, the "agent for suppressing the expression of a target gene" refers to an agent having the activity of suppressing the expression of a target gene. In general, the agent refers to a substance or composition (mixture) which comprises as an active ingredient a DNA or vector of the present invention, which is used to suppress the expression of a target gene in plants or seeds thereof.

Herein, the "agent for inducing target gene silencing" refers to an agent having the activity of inducing target gene silencing. In general, the agent refers to a substance or composition (mixture) which comprises as an active ingredient a DNA or vector of the present invention, which is used to induce the silencing of a target gene in plants or seeds thereof.

The present invention also provides methods for producing expression vectors for siRNAs. Specifically, the present invention provides methods for producing expression vectors for siRNAs, which comprise the steps of:
(1) operably linking a DNA encoding RSIS to:
   (a) a DNA that encodes a promoter sequence upstream of a DNA encoding RSIS;
   (b) a DNA that encodes a terminator sequence downstream of a DNA encoding RSIS; and
   (c) a DNA that encodes the full-length or a partial sequence of target gene mRNA, downstream of the DNA of (a), and upstream of the DNA of (b); and
(2) inserting the DNA into a vector.

In addition, the present invention also provides methods for producing expression vectors for siRNAs, which comprise the steps of:
(1) operably linking a DNA encoding RSIS to:
   (a) a DNA that encodes a promoter sequence upstream of a DNA encoding RSIS;
   (b) a DNA that encodes a terminator sequence downstream of a DNA encoding RSIS; and
   (c) a DNA that encodes the full-length or partial sequences of mRNAs of multiple target genes, downstream of the DNA of (a), and upstream of the DNA of (b); and
(2) inserting the DNA into a vector.

The DNAs of (a) to (c) in the above-described production methods may be DNAs described above. The insertion in step (2) of the production method described above can be appropriately achieved according to the description on the vectors described above.

Furthermore, the present invention relates to the use of DNAs or vectors of the present invention in manufacturing agents for suppressing the expression of target genes or agents for inducing target gene silencing.

All prior art documents cited in the specification are incorporated by reference herein.

### Examples

The present invention is specifically illustrated below with reference to Examples, but it is not to be construed as being limited thereto.

The materials and methods used in the experiments are described below in (1) to (4).
(1) Preparation of constructs
   Genes were digested into fragments and the fragments were ligated by conventional methods using restriction enzymes and DNA ligase. Genomic sequences (fragments) derived from rice (varieties: Kita-ake and Nipponbare), except those already cloned, were newly cloned by amplifying gene fragments using PCR with primers designed for the two ends of each gene.
   The mGLP sequence (SEQ ID NO: 2) and RSIS (RNA silencing inducible sequence) sequence (SEQ ID NO: 3), which are used as the backbone for inducing RNA silencing, are shown in Fig. 1. The mGLP sequence is a sequence obtained by substituting Ser for Ala at position 2 in the nucleotide sequence of GLP-1, a human peptide hormone, and replacing its codons with cordons that appear frequently in rice endosperm (Fig. 1). The RSIS sequence is a sequence obtained by replacing restriction sites (*Eco*RI and *Xho*I) in the mGLP sequence.
   The promoter and sequence of the 5' end of mRNA (about 100 bp) of a target gene were linked upstream of the sequences described above, and the terminator sequence after the stop codon was linked downstream of the sequences described above. The resulting constructs thus prepared were transformed into rice plant (Kita-ake) by the Agrobacterium method. The constructs used in the transformation are specifically described in the Description of the Drawings.
(2) SDS-PAGE and Western blotting
   Total protein of rice seed was prepared by crushing each grain of mature seeds from the respective transformants with a multi-beads shocker, and then vigorously stirring the crushed seeds in 500 µl of protein extraction buffer [8 M urea, 4%(w/v) SDS, 5%(v/v) 2-mercaptoethanol, 20%(w/v) glycerol, 20 mM Tris-HCl (pH 6.8)] for one hour.
   SDS-PAGE was carried out by Laemmuli's method (Laemmuli UK. Cleavage of structural proteins during the assembly of the heads of bacteriophase T4. Nature 227; 680-685 (1970)) using 12% get. 2 µl aliquots of the protein extracts were subjected to SDS-PAGE.
   Western blotting was carried out by electrotransfer of the gel onto PVDF membrane after SDS-PAGE. The reaction between each peptide and a specific antibody was induced by the method ofYasuda et al. (Yasuda H., Tada Y., Hayashi Y., Jomori T., and Takaiwa F. Expression of the small peptide GLP-1 in transgenic plants. Transgenic Res. 14; 677-684 (2005)). The detection was carried out using ECL Detection kit (Amersham) according to the manual.
   To detect oleosin protein, mature seeds were dissected into embryos and endosperms, and then total protein was extracted. In this experiment, total embryonic protein was extracted using 100 µl of protein extraction buffer for each embryo. The 10-µl aliquots were subjected to SDS-PAGE and Western blotting. The total endospermic protein was prepared by the same method as described above.
(3) RNA preparation and expression analysis by Northern blotting or RT-PCR
   Total RNA was extracted from seeds 5, 7, 10, 15, 20, and 25 days after flowering by the method of Yasuda *et al.* (Yasuda H., Tada Y., Hayashi Y., Jomori T., and Takaiwa F. Expression of the small peptide GLP-1 in transgenic plants. Transgenic Res. 14; 677-684 (2005)).
   2 µg of total RNA was subjected to Northern blotting using 1.2% agarose gel. Then, the RNA was transferred onto Hybond-N+ (Amersham). Then, an RI-labeled GluB1 probe was hybridized, and detected by autoradiography.
   Small RNA was prepared, electrophoresed, and transferred onto Hybond-N+ according to the method of Mette *et al.* (Mette M. F., Aufsatz W., van der Winden J., Matzke M. A., and Matzke A. J. M. Transcriptional silencing and promoter methylation triggered by double-stranded RNA. EMBO J. 19; 5194-5201 (2000)). The mGLP sequence was used as a gene transfer-specific probe, while the GluB1 terminator sequence was used as a GluB1-specific probe. Then, each RI-labeled probe was hybridized, and detected by autoradiography.
   The CYP90D2 gene was detected by RT-PCR. Total RNA was prepared from a leaf of a plant regenerated from a transformant using RNeasy Plant Mini Kit (QIAGEN) according to the manual. cDNA was synthesized by reverse transcription of the obtained total RNA using SuperScript^{™}III RNaseH⁻ Reverse Transcriptase (Invitrogen) and sequence-specific antisense primers (actin and CYP90D2 genes) according to the manual. PCR was carried out using the prepared cDNA as a template and sequence-specific primers to amplify and detect the genes of interest.
(4) Assessment of RNA silencing

When endosperms are tested for the induction of RNA silencing, it should be noted that the seeds are a heterologous population because they are already in subsequent generations. In RNA silencing using RSIS, the phenotype is known to be expressed in a dominant fashion. Thus, at least four grains of seed were tested for each transformant, and when silencing was confirmed in one or more grains, RNA silencing was judged to occur in the plant. The RNA silencing efficiency was assessed in terms of the ratio of "the number of plants in which the silencing was judged to be induced" against "the total number of the regenerated plants tested".

The silencing of the CYP90D2 gene was tested by RT-PCR using total RNA from leaves of regenerated plants. When no amplification of the CYP90D2 gene was observed, RNA silencing was judged to be induced in the plant.

### [Example 1] RNA silencing using the mGLP sequence

### (1-1) RNA silencing using construct 1 (2.3-k pGluB1-5'UTR-SP-mGLP (KDEL)-GluB1 3'UTR-terminator)

The 5'UTR of GluB1, signal peptide, and the mGLP sequence were linked downstream of the 2.3-k GluB1 promoter, an endosperm-specific overexpression promoter, and then a terminator sequence including the 3'UTR of GluB1 was linked downstream thereof. The construct (Fig. 2A; Construct 1) thus prepared was introduced into rice plants.

Neither transcripts nor translation products originated from the mGLP sequence were detected (data not shown). Total protein was extracted from the yielded seeds, and subjected to SDS-PAGE. The result showed that some of bands for glutelin, a rice storage protein, were missing and 13-kD prolamin was increased (Fig. 2C).

Then, Western analysis was carried out using an anti-GluB antibody. The result showed that the disappearance of the glutelin bands was ascribed to the disappearance of the GluB family (GluB1, GluB2, and GluB4) (Fig. 2C).

Furthermore, Northern analysis was carried out using a GluB1 gene probe. No GluB1 gene transcript was detected (Fig. 2B).

The results of SDS-PAGE and Western analysis suggested that the Expression of genes of the GluB family was suppressed at the transcription level. Thus, the GluB1 gene, which was linked to upstream and downstream of the mGLP sequence, was assumed to be silenced in rice plants introduced with Construct 1.

### (1-2) Expression levels of genes, each of which has a different homology to the GluB1 gene, in rice plants introduced with Construct 1

The expression of the GluB family, which is highly homologous to the GluB1 gene, was suppressed in rice plants introduced with Construct 1. Accordingly, Northern analysis was carried out to assess the expression of the GluA2 gene, which is highly homologous to the GluB1 gene; the GluC gene, which belongs to the same glutelin family but has almost no homology to the GluB1 gene; and the gene for13-kD prolamin, which is a seed storage protein.

The result showed that the transcript of the GluA2 gene, which has high homology to the GluB1 gene, was decreased (Fig. 3A). The expression levels of low-homologous GluC and 13-kD prolamin genes were comparable to those in non-recombinant plants (Fig. 3A).

### (1-3) Detection of siRNA in rice plants introduced with Construct 1

The finding that the expression of homologous genes is suppressed increases the possibility that the suppression was caused by RNA silencing. Thus, siRNA (small interfering RNA) detection was carried out to obtain evidence for RNA silencing.

As a result, some bands were detected using introduced-gene-specific probe (mGLP) and GluB1-specific probe (GluB1 terminator) (Fig. 3B). This demonstrates that RNA silencing occurred in rice plants introduced with Construct 1.

### (1-4) mGLP sequence-mediated suppression of prolamin gene expression

Next, to assess whether RNA silencing for not only GluB1 but also other genes (10-kDa and 16-kDa prolamins) was induced by the mGLP sequence, some constructs (Fig. 4A; Constructs 2 and 3) were prepared and introduced into rice plants. Total protein was prepared from seeds of each transformant yielded, and analyzed by Western blotting.

The result showed that the expression of 10-kDa and 16-kDa prolamins was suppressed (Fig. 4B). This suggests that the mGLP sequence can induce RNA silencing for not only the GluB1 gene but also other genes.

### [Example 2] Analysis of the mGLP sequence that induces RNA silencing

Various constructs (Fig. 5) were prepared to assess whether the mGLP sequence induces RNA silencing. The relationship between the mGLP sequence and RNA silencing was evaluated.

First, a construct (Fig. 5; Construct 0) was prepared by deleting the mGLP sequence from Construct 1, and then introduced into rice plants. There was no plant line in which GluB1 expression was suppressed (Table 1; the efficiency of silencing in rice endosperm introduced with the construct shown in Fig. 5).

**Table 1**

| | CONSTRUCT | TARGET | EFFICIENCY |
|---|---|---|---|
| CONSTRUCT 0 | 2.3k GluB1 PROMOTER -5'UTR-SP-GluB1 3'UTR-TERMINATOR | GluB | 0/25 (0%) |
| FRAME-SHIFT | 2.3k GluB1 PROMOTER -5'UTR-SP-+G-mGLP(KDEL)-GluB1 3'UTR- TERMINATOR | GluB | 5/14 (35.7%) |
| GFP (630-720) | 2.3k GluB1 PROMOTER -5'UTR-SP-GFP(630-720-KDEL)-GluB1 3'UTR- TERMINATOR | GluB | 0/16 (0%) |
| mGLP(1-45) | 2.3k GluB1 PROMOTER -5'UTR-SP-mGLP(1-45-KDEL)-GluB1 3'UTR-TERMINATOR | GluB | 0/11 (0%) |
| mGLP(46-90) | 2.3k GluB1 PROMOTER -5'UTR-SP-mGLP(46-90-KDEL)-GluB1 3'UTR-TERMINATOR | GluB | 2/28 (7.1%) |
| mGLP×2 | 2.3k GluB1 PROMOTER -5'UTR-SP-mGLP×2(KDEL)-GluB1 3'UTR-TERMINATOR | GluB | 5/16 (31.3%) |
| mGLP×3 | 2.3k GluB1 PROMOTER -5'UTR-SP-mGLP×3(KDEL)-GluB1 3'UTR-TERMINATOR | GluB | 0/11 (0%) |

Next, a frame-shift construct (Fig. 5; frame-shift) was prepared by inserting a guanine residue before the mGLP sequence to inhibit the expression of a translation product derived from the mGLP sequence, because there was a possibility that the translation product of the mGLP sequence induced the silencing. As a result of gene transfer of the prepared construct, the expression of the GluB family was suppressed in about 30% of the yielded lines (Table 1). This suggests that the translation product derived from the mGLP sequence was not involved in the induction of RNA silencing.

Furthermore, a construct (Fig. 5; GFP(630-720)) having the same length (90 bp) but a different sequence was prepared and introduced, because there was a possibility that the silencing induction depended on the length of transcript of the mGLP sequence. The different sequence used was the C-terminal 90-bp portion of GFP, which has been commonly used as a reporter gene. There was no plant line in which GluB1 expression was suppressed (Table 1). This suggests that the induction of RNA silencing does not depend on the length of a transcript of the mGLP sequence.

In order to reveal which portion of the mGLP sequence is responsible for the induction of silencing, some constructs (Fig. 5; mGLP(1-45) and mGLP(46-90)) were prepared by deleting the last or first half of the mGLP sequence, and introduced into rice plants. The result showed that silencing for the GluB family was found in only two rice plants (about 7%) introduced with mGLP(46-90) but not in the other plants (Table 1). This suggests that the entire mGLP sequence is necessary for the efficient induction of silencing.

Furthermore, constructs (Fig. 5; mGLPx2 and mGLPx3) having two or three units of the mGLP sequence were prepared and introduced into rice plants. The silencing for the GluB family was detected in about 30% of lines introduced with the construct having two units of the mGLP sequence (mGLPx2), while the silencing was not detected in the lines introduced with the construct having three units of the mGLP sequence (mGLPx3) (Table 1). The translation product of mGLPx3 (mGLPx3 peptide) was found to be accumulated in the rice plants introduced with mGLPx3 (data not shown).

This suggests that the mGLP sequence has a conformation required to induce RNA silencing and the conformation remains to some extent even when two units of the sequence are linked together. However, the conformation was assumed to be destroyed when three units of the sequence are linked together.

### [Example 3] mGLP sequence-mediated RNA silencing for multiple genes

### (3-1) mGLP sequence-mediated RNA silencing for the glutelin and globulin genes

Next, the present inventors aimed at RNA silencing for multiple genes using the mGLP sequence. A construct (Fig. 6A; Construct 4) was prepared by linking the mGLP sequence downstream of the GluB1 promoter, 5'UTR, and signal peptide, and linking a terminator sequence including the 3'UTR of globulin at the C terminus thereof. Total protein was extracted from seeds of rice plants introduced with the construct, and subjected to SDS-PAGE.

The result showed that the bands of glutelin and globulin disappeared while the amount of accumulated 13-kD prolamin increased (Fig. 6B).

Western analysis was carried out using the respective specific antibodies, and the result showed that both proteins completely disappeared (Fig. 6B). This suggests that RNA silencing for multiple genes can be achieved by using the mGLP sequence.

### (3-2) RSIS sequence-mediated RNA silencing for the prolamin gene

It has been reported that there are several tens or more copies of 13-kD prolamin in the rice plant genome (Kim W. T. and Okita T. W. Structure, expression and heterogeneity of the rice seed prolamins. Plant Physiol. 88; 649-655 (1988)). Accordingly, the present inventors aimed at suppressing the expression of 13-kD prolamin gene which has a number of copies.

To achieve the silencing of the 13-kD prolamin family, a construct (Fig. 7A; Construct 5) was prepared by linking RSIS sequence (Fig. 1) downstream of the RM1 (Cys-rich type prolamin) promoter, 5'UTR, and signal peptide, and linking a terminator sequence including the 3'UTR of RM2 (Cys-poor type prolamin) at the C terminus thereof The resulting construct was introduced into rice plants. Total protein was extracted from seeds of the prepared transformants, and subjected to SDS-PAGE.

The result showed that the amount of accumulated 13-kD prolamin was markedly reduced (Fig. 7B). This suggests that RSIS can induce RNA silencing even for genes having a number of copies.

### [Example 4] Intercellular signaling of mGLP sequence-mediated RNA silencing

Next, the present inventors assessed whether the signal of mGLP sequence-mediated RNA silencing could be transduced between cells and the effect could spread. The oleosin gene has been reported to be expressed in the rice seed embryos and aleurone cells (Wu L. S. H., Wang L.-D., Chen P.-W., Chen L.-J. and Tzen J. T. C. Genomic cloning of 18 kDa oleosin and detection oftriacylglycerols and oleosin isoforms in maturing rice and postgerminative seedling. J. Biochem. 123; 386-391 (1998)). Thus, a construct (Fig. 8A; Construct 6) was prepared by linking the mGLP sequence downstream of the oleosin promoter and 5'UTR, and linking a terminator sequence including the 3'UTR of oleosin at the C terminus thereof. Another construct (Fig. 8A; Construct 7) was prepared by linking a terminator sequence including the mGLP sequence and oleosin 3'UTR downstream of the GluB1 promoter, 5'UTR, and signal peptide. The resulting constructs were introduced into rice plants. Total protein was extracted from embryos and endosperms of each transformant yielded, and analyzed by Western blotting using an anti-oleosin antibody.

The result showed that the expression of oleosin was suppressed in both embryo and endosperm of transformants in which the silencing had been induced under the control of the oleosin promoter (Construct 6) (Fig. 8B), By contrast, oleosin was detectable in the embryo but not in the endosperm of transformants in which the silencing had been induced under the control of the endnsperm-specific GluB promoter (Construct 7) (Fig. 8B).

These findings suggest that, in mGLP sequence-mediated RNA silencing, the endospermic silencing signal does not spread to the embryo, and thus the signal is not transduced between cells. This result raises the possibility that the expression of a target gene can be suppressed only in the tissue of interest.

### [Example 5] Induction of RNA silencing in non-seed tissues by RSIS

The induction of silencing in rice seeds was assessed as described above. Next, the present inventors assessed whether RSIS could induce RNA silencing also in other tissues.

The CYP90D2 gene has been reported to be a novel gene for brassinosteroid biosynthesis enzyme, which belongs to the family cytochrome P450 (Hong Z., Ueguchi-Tanaka M., Umemura K., Uozu S., Fujioka S., Takatsuto S., Yoshida S., Ashikari M., Kitano H. and Matsuoka M. A rice brassinosteroid-deficient mutant, ebisu dwarf (d2), is caused by a loss of function of a new member of cytochrome P450. Plant Cell 15; 2900-2910 (2003)).

To suppress the expression of the gene, a construct (Fig, 9A; Construct 8) was prepared by linking a terminator sequence including RSIS and CYP90D2 3'UTR downstream of the 35S promoter. The construct was introduced into rice plants. Total RNA was extracted from the leaves of regenerated plants, and tested for the expression of the CYP90D2 gene by RT-PCR.

The result showed that the expression of the CYP90D2 gene was not detected in 26 of 40 regenerated plants and thus the silencing was induced with a frequency of about 65% (Fig. 9B) (Table 2: the efficiency of mGLP sequence-mediated silencing).

### [Example 6] RSIS-mediated suppression of constitutively expressed genes

The 33-kDa allergen gene is relatively constitutively expressed in rice plants. To suppress the expression of this gene, a construct (Fig. 11A; Construct 9) was prepared by linking a terminator sequence including the 5'UTR, RSIS, and 3'UTR of 33-kDa allergen downstream of the promoter of the 33-kDa allergen gene. The construct was introduced into rice plants. Total RNA was extracted from leaves, stems, and seeds of the regenerated plants, and tested for the expression of the 33-kDa allergen gene by RT-PCR.

The result showed that the expression of the 33-kDa allergen gene was suppressed in 8 of 16 regenerated plants, and thus the silencing was induced with a frequency of 50% (Fig. 11B) (Table 2: the efficiency of mGLP sequence-mediated silencing).

**Table 2**

| | CONSTRUCT | TARGET | EFFICIENCY |
|---|---|---|---|
| CONSTRUCT 1 | 2,3k GluB1 PROMOTER -5'UTR-SF-mGLP(KDEL)-GluB13'UTR-TERMINATOR | GluB | 14/24 (54.2%) |
| CONSTRUCT 2 | 10kDa PROLAMIN PROMOTER -5'UTR-SP-mGLP(KDEL)-10kDaPROLAMIN 3'UTR-TERMINATOR | 10kDa PROLAMIN | 6/15 (40.0%) |
| CONSTRUCT 3 | 16kDa PROLAMIN PROMOTER-5'UTR-SP-mGLP(KDEL)-16kDa PROLAMIN 3'UTR-TERMINATOR | 16kDa PROLAMIN | 12/28 (42.8%) |
| CONSTRUCT 4 | 2.3k GluB1 PROMOTER-5'UTR-SP-mGLP(KDEL)-GLOBULIN 3'UTR-TERMINATOR | GluB GLOBULIN | 6/15 (40.0%) |
| CONSTRUCT 5 | RM1 PROMOTER -5'UTR-SP-RSIS-RM2 3'UTR-TERMINATOR | RM1 RM2 | 17/29 (58.6%) |
| CONSTRUCT 6 | 18kDa OLEOSIN PROMOTER-3'UTR-SP-mGLP(KDEL)-18kDa OLEOSIN3'UTR-TERMINATOR | 18kDa OLEOSIN | 7/12 (58.3%) |
| CONSTRUCT 7 | 2.3k GluB1 PROMOTER-5'UTR-SP-mGLP(KDEL)-18kDa OLEOSIN 3'UTR- TERMINATOR | GluB 18kDa OLEOSIN | 4/11 (36.6%) |
| CONSTRUCT 8 | 35S PROMOTER-RSIS-CYP90D2 3'UTR-TERMINATOR | CYP90D2 | 26/40 (65%) |
| CONSTRUCT 9 | 33kDa ALLERGEN PROMOTER-5'UTR-RSIS-33kDa 3'UTR-TERMINATOR | 33kDa ALLERGEN | 8/16 (50%) |

This raises the possibility that the RSIS-mediated silencing is not specific to rice seeds but can be induced in the whole rice plant.

### [Example 7] Efficiency of mGLP sequence-mediated silencing

The efficiencies of mGLP sequence-mediated RNA silencing and RSIS-mediated DNA silencing are summarized above in Table 2.

The efficiency for the induction of mGLP sequence-mediated RNA silencing is 40% or more, and is assumed to be practically high enough to use in studies using RNA silencing. The efficiency is comparably high to those of the antisense methods and the silencing based on the hairpin structure.

### Industrial Applicability

In conventional methods for suppressing gene expression, sequences of sense- and antisense-orientation have to be linked together in a single construct so as to form an inverted repeat sequence or hairpin-loop structure, and such procedures are complicated. They become more complicated when the expression of multiple genes is to be suppressed at the same time.

The RSIS-mediated methods for suppressing gene expression or inducing gene silencing discovered this time by the present inventors are very simple and convenient, because they can suppress the expression of genes of interest or induce silencing of genes of interest merely by linking a portion of a gene of interest (target gene) to either one end or both ends of RSIS. Furthermore, even when there are multiple genes of interest, the methods are very simple compared to conventionally used complicated procedures, because the methods of the present invention can suppress the expression of genes of interest or induce silencing of genes of interest at the same time, simply by linking a portion of each of the multiple genes of interest to either one end or both ends of RSIS.

RNA silencing has been mainly used to elucidate gene function. Recently, however, RNA silencing is also used to produce practical plant varieties, for example, to alter flower color.

The present inventors envision the RNA silencing-based strategy shown in Fig. 10. Rice endospermie tissues can accumulate seed storage proteins in high amounts, i.e. in amounts equivalent to about 6% to 8% of the live weight % of seeds (rice). This suggests that (1) the rice endosperm has the mechanism to express such a large amount of protein; (2) it can supply amino acids, which are precursor substances of protein; and (3) it has the ability to secure the sites (protein bodies) for accumulating synthesized peptide. This shows that the rice endosperm has superior characteristics of a so called "plant factory" capable of high expression and high accumulation of foreign gene products.

Glutelin accounts for about 80% of rice seed storage proteins. If the amount of accumulated glutelin can be minimized so that foreign gene products can be accumulated in larger amounts, mechanisms required to accumulate foreign gene products can be maximally secured.

Thus by preparing a construct for reducing the amount of accumulated endogenous glutelin and such based on RNA silencing (Fig. 10(1)), and another construct for expressing a gene of interest under the control of endosperm-specific high-accumulation promoter (Fig. 10(2)), and then introducing the two constructs into rice at the same time, the accumulation is expected to be more than when the gene of interest is expressed alone.

Furthermore, it is known that the amount of accumulated prolamin increases with the decrease in the accumulated amount of glutelin. Accordingly, when a construct for expressing a gene of interest fused with the prolamin gene (Fig. 10(2)) is co-introduced with the glutelin-sileneing construct (Fig. 10(1)) into rice plants, genetic recombinant rice plants carrying the gene product of interest accumulated in a higher amount is expected to be produced.

The present inventors believe that a gene product of interest can be accumulated in a higher amount by using an appropriate combination of a silencing construct with a constructs for expressing a gene of interest. Furthermore, when multiple constructs are introduced, time and effort can be reduced by using the MultiSide Gateway System (Wakasa Y., Yasuda H. and Takaiwa F. High accumulation of bioactive peptide in transgenic rice seeds by expression of introduced multiple genes. Plant Biotechnol. J. 4; 499-510 (2006)), as compared to crossing methods.

Meanwhile, a number of seed proteins can act as allergens in rice allergy. Thus, the RSIS-mediated methods of the present invention for inducing the silencing can be used to efficiently suppress the expression of multiple antigen proteins. Thus the methods of the present invention are expected to be applicable for producing low-allergen or allergen-free rice.

Furthermore, when gene expression knockdown plant lines are produced by RNA silencing using the RSIS sequence of the present invention, the accumulation of metabolites of interest can be increased by specifically regulating the metabolic pathway. Alternatively, gene expression can be efficiently suppressed by applying the RSIS sequence to a consensus gene sequence among multigenes, while the expression of a specific gene can be efficiently suppressed by applying the RSIS sequence to the 5'- or 3'-untranslated region. In such methods, the suppression can be achieved by linking to either or both of the untranslated regions. A maximum of about eight genes can be specifically suppressed by using the Gateway method.

Alternatively, when the RSIS sequence of the present invention is inserted or introduced by substitution into the coding regions of constitutively expressed genes to be suppressed or to be controlled, the expression of the genes can be specifically suppressed. Indeed, the expression of the 33-kDa allergen gene in tissues expressing the gene could be specifically suppressed when the RSIS sequence was substituted or inserted into the coding region of the 33-kDa allergen gene, which is a single copy gene in the genome and expressed in leaves, stems, and seeds, and then introduced into rice plants.

## Claims

1. A DNA of any one of:
(a) a DNA comprising the nucleotide sequence of SEQ ID NO: 3;
(b) a DNA that hybridizes under stringent conditions to a DNA comprising the nucleotide sequence of SEQ ID NO: 3 and is functionally equivalent to the DNA comprising the nucleotide sequence of SEQ ID NO: 3; and
(c) a DNA comprising a nucleotide sequence having a substitution, deletion, addition, and/or insertion of one or more nucleotides in the nucleotide sequence of SEQ ID NO: 3.

2. A DNA that suppresses the expression of a target gene, which has a structure in which the DNA of claim 1 is operably linked to:
(a) a DNA encoding a promoter sequence upstream of the DNA of claim 1;
(b) a DNA encoding a terminator sequence downstream of the DNA of claim 1; and
(c) a DNA encoding the full-length or a partial mRNA of the target gene downstream of the DNA of (a) and upstream of the DNA of (b).

3. A DNA that suppresses the expression of multiple target genes at the same time, which has a structure in which the DNA of claim 1 is operably linked to:
(a) a DNA encoding a promoter sequence upstream of the DNA of claim 1;
(b) a DNA encoding a terminator sequence downstream of the DNA of claim 1; and
(c) a DNA encoding the full-length or a partial mRNA of each of the multiple target genes downstream of the DNA of (a) and upstream of the DNA of (b).

4. A protein encoded by the DNA of any one of claims 1 to 3.

5. A vector carrying the DNA of any one of claims 1 to 3.

6. A composition comprising the DNA of any one of claims 1 to 3 or the vector of claim 5.

7. A transformed plant cell introduced with the DNA of any one of claims 1 to 3 or the vector of claim 5.

8. A transformed plant comprising the transformed plant cell of claim 7.

9. A transformer plant, which is a progeny or clone of the transformed plant of claim 8,

10. A breeding material of the transformed plant of claim 8 or 9.

11. A method for suppressing the expression of a target gene, which uses the DNA of any one of claims 1 to 3 or the vector of claim 5.

12. A method for inducing the silencing of a target gene, which uses the DNA of any one of claims 1 to 3 or the vector of claim 5.

13. A method tor producing a transformed plant, which comprises the step of introducing a plant cell with the DNA of any one of claims 1 to 3 or the vector of claim 5 and regenerating a plant from the plant cell.

14. A method for producing a plant in which the expression of a target gene is suppressed, or a seed thereof, which comprises the step of expressing the DNA of any one of claims 1 to 3 or the vector of claim 5 in a cell of the plant.

15. An agent for suppressing the expression of a target gene, which comprises as an active ingredient the DNA of any one of claims 1 to 3 or the vector of claim 5.

16. An agent for inducing the silencing of a target gene, which comprises as an active ingredient the DNA of any one of claims 1 to 3 or the vector of claim 5.

17. A method for producing an siRNA expression vector, which comprises the steps of:
(1) operably linking the DNA of claim 1 to:
(a) a DNA encoding a promoter sequence upstream of the DNA of claim 1;
(b) a DNA encoding a terminator sequence downstream of the DNA of claim 1; and
(c) a RNA encoding the full-length or a partial mRNA of the target gene downstream of the DNA of (a) and upstream of the DNA of (b); and
(2) inserting the DNA into a vector.

18. A method for producing an siRNA expression vector, which comprises the steps of:
(1) operably linking the DNA of claim 1 to:
(a) a DNA encoding a promoter sequence upstream of the DNA of claim 1;
(b) a DNA encoding a terminator sequence downstream of the DNA of claim 1; and
(c) a DNA encoding the full-length or a partial mRNA of each of the multiple target genes downstream of the DNA of (a) and upstream of the DNA of (b); and
(2) inserting the DNA into a vector.


SEQUENCE LISTING

<110>  NATIONAL INSTITUTE OF AGROBIOLOGICAL SCIENCES

<120>  Convenient method for inhibition of gene expression using RSIS

<130>  MOA-A0606P

<150>  JP 2007-008994
<151>  2007-01-18

<160>  5

<170>  PatentIn version 3.3

<210>  1
<211>  90
<212>  DNA
<213>  Homo sapiens

<400>  1
catgctgaag ggacctttac cagtgatgta agttcttatt tggaaggcca agctgccaag      60

gaattcattg cttggctggt gaaaggccga                                      90


<210>  2
<211>  90
<212>  DNA
<213>  Artificial

<220>
<223>  An artificially synthesized nucleotide sequence

<400>  2
cattctgagg gaactttcac atctgatgtt agttcttacc tcgagggcca agcagctaag      60

gaattcatcg cttggctcgt aaagggccgt                                      90


<210>  3
<211>  90
<212>  DNA
<213>  Artificial

<220>
<223>  An artificially synthesized nucleotide sequence

<400>  3
gattctgagg gaactttcac atctgatgtt agttcttacc tcgcgggcca agcagctaag      60

gatttcatcg cttggctcgt aaagggccgt                                      90


<210>  4
<211>  44
<212>  DNA
<213>  Oryza sativa

<400>  4
tcacatcaat tagcttaagt ttccataagc aagtacaaat agct                      44


<210>  5
<211>  75
<212>  DNA
<213>  Oryza sativa

<400> 5
atggcgagtt ccgttttctc tcggttttct atatactttt gtgttcttct attatgccat 60

ggttctatgg cccag 75